# EUROPÄISCHE PATENTSCHRIFT

(11) **EP 4 283 260 B1**
(45) Veröffentlichungstag und Bekanntmachung des Hinweises auf die Patenterteilung: **11.11.2026**
(21) Anmeldenummer: 22175769.3
(22) Anmeldetag: 27.05.2022
(51) Int. Cl.: G01F 11/08

(54) **BEHÄLTER ZUR DOSIERTEN ABGABE EINER FLÜSSIGKEIT**
CONTAINER FOR DOSED DISPENSING OF A LIQUID
RÉCIPIENT DESTINÉ À LA DISTRIBUTION DOSÉE D'UN LIQUIDE

(43) Veröffentlichungstag der Anmeldung: 29.11.2023
(73) Patentinhaber: Packsys GmbH, 82340 Feldafing/OT Wieling (DE)
(72) Erfinder: Burkhardt, Sybille, 73466 Lauchheim (DE); Thiermeyer, Josef, 82418 Murnau (DE); Lössl, Veronika, 83026 Rosenheim (DE); Finger, Markus, 82319 Starnberg (DE)
(74) Vertreter: Hoffmann Eitle

(56) Entgegenhaltungen:
- EP-A1- 0 972 722
- EP-A1- 2 316 739
- WO-A1-2013/163364
- DE-A1- 2 755 112
- DE-A1- 2 802 893
- DE-A1- 3 716 822
- DE-B1- 1 708 552
- FR-A- 1 068 919
- FR-A1- 2 568 007

## Beschreibung

### Gebiet der Erfindung

Die Erfindung betrifft einen Behälter zur dosierten Abgabe einer sterilen Flüssigkeit.

### Hintergrund der Erfindung

Sterile Flüssigkeiten, wie beispielsweise Augentropfen, werden entweder in Einzeldosen angeboten oder aber aus Behältern abgegeben, welche die Menge an steriler Flüssigkeit für eine Mehrfachanwendung beinhalten. Augentropfen werden vom Anwender in den Bindehautsack oder auf die Hornhaut getropft und müssen steril sein, um Augeninfektionen zu vermeiden.

Wenn sterile Flüssigkeiten in genau vorgeschriebenen Dosen in Form einer gewissen Anzahl von Tropfen auszugeben sind, wurden im Stand der Technik Behälter vorgeschlagen, mit denen sich eine definierte Teilmenge aus dem Behälter abgeben lassen. Dazu ist der Vorratsbehälter beispielsweise mit einer elastischen Seitenwand versehen, die bei der Anwendung vom Benutzer zusammengedrückt werden kann, um eine oder mehrere Einzeltropfen ausgeben zu können. Dabei wird der Behälter bei der Abgabe von Augentropfen in der Regel über Kopf gestellt, damit die ausgegebenen Tropfen im Wesentlichen von oben in das Auge eines Benutzers gelangen.

Im Stand der Technik wurden auch Behälter vorgeschlagen, deren Bodenwand zwei definierte Zustände einnehmen kann. Durch Druck auf den Boden kann ein Behälterboden, der in der ersten Position in Richtung auf die Außenseite des Behälters gewölbt ist, durch Druckbeaufschlagung auf die Bodenwand in eine zweite Position überführt werden, in welcher der Boden in Richtung auf das Innenvolumen des Behälters gewölbt ist.

Bei der Bewegung von der ersten Position in die zweite Position wird ein definiertes Volumen in Inneren des Behälters verdrängt, wodurch eine definierte Flüssigkeitsmenge aus einer Abgabeöffnung ausgegeben werden kann.

Dosierbehälter, die bereichsweise die Form eines Faltenbalgs besitzen, sind aus der FR 2 568 007 A1, WO 2013/163364 A1 und DE 27 55 112 A1 bekannt.

Die FR 1 068 919 A1 stellt den nächstkommenden Stand der Technik dar.

Da die Funktion eines derartigen Bodens, der verschiedene geometrische Zustände einnehmen kann, für einen Benutzer nicht intuitiv erkennbar ist, besteht ein Bedürfnis nach einem Behälter zur dosierten Abgabe einer sterilen Flüssigkeit, dessen Betätigung für einen durchschnittlichen Benutzer bereits aus dessen Formgebung erkennbar und ableitbar ist. Gleichzeitig muss der Behälter einfach gefertigt werden können, da es sich bei derartigen Behältern um Massenprodukte handelt.

### Darstellung der Erfindung

Diese Aufgabe wird durch einen Behälter zur dosierten Abgabe einer sterilen Flüssigkeit mit den Merkmalen des Anspruchs 1 gelöst. Bevorzugte Ausführungsformen folgen aus den übrigen Ansprüchen.

Ein erfindungsgemäßer Behälter zur dosierten Abgabe einer sterilen Flüssigkeit umfasst einen im Wesentlichen rotationssymmetrischen Aufbewahrungskörper aus Kunststoff mit einer Längsachse und einer senkrecht zur Längsachse verlaufenden radialen Richtung, wobei der Aufbewahrungskörper einen im Wesentlichen zylinderförmigen oder kegelförmigen Mantelabschnitt mit einer ersten Wandstärke, einen Dosierabschnitt, einen Bodenabschnitt und einen Behälterhalsabschnitt umfasst. Der Behälter ist dadurch gekennzeichnet, dass der Dosierabschnitt zwischen dem Mantelabschnitt und dem Bodenabschnitt angeordnet ist und als Faltenbalg mit einer einzigen Faltung radial nach innen mit Gelenkstellen ausgestaltet ist, wobei die Wandstärke des Dosierabschnitts zumindest im Bereich der Gelenkstellen um 25% bis 60% gegenüber der ersten Wandstärke reduziert ist.

Der erfindungsgemäße Behälter weist somit einen zwischen dem Mantelabschnitt und dem Bodenabschnitt des Behälters angeordneten Dosierabschnitt auf, der gut erkennbar im Bereich der Mantelfläche des Behälters angeordnet ist. Dieser Dosierabschnitt ist als Faltenbalg umfassend Gelenkstellen ausgestaltet, wodurch die Einfaltung radial nach innen für einen Benutzer gut sichtbar ist. Daher erkennt ein Benutzer auch intuitiv, wie der erfindungsgemäße Behälter anzuwenden ist. Die Anwendung wird dadurch ermöglicht, dass die Wandstärke des Dosierabschnitts zumindest im Bereich der Gelenkstellen der Faltung mit einer verringerten Wandstärke versehen ist, wobei die Wandstärkte zumindest im Bereich der Gelenkstellen um 25% bis 60% gegenüber der Wandstärke des im Wesentlichen zylinderförmigen oder kegelförmigen Mantelabschnitts reduziert ist. Durch die verringerte Wandstärke der Gelenkstellen lässt sich der einstückig hergestellte Behälter im Bereich der Faltung zusammenfalten, indem sich bei einem Druck auf den Bodenabschnitt die Höhe des Behälters verringert, weil die Höhe des Dosierabschnitts verringert wird. Die verringerte Wandstärke ist dabei vorzugsweise nur im Bereich der Gelenkstellen vorgesehen, wodurch nur wenige Stellen mit geringer Wandstärke gebildet sind, die in Bezug auf das Eindringen beispielsweise von Sauerstoff durch Diffusion durch die Behälterwand auch im Dosierabschnitt gering gehalten werden kann. Dies ist vorteilhaft, weil insbesondere bei sauerstoffempfindlichen Produkten das Stabilitätsverhalten durch eine großflächige Reduzierung der Wandstärke verringert werden würde. Da der Dosierabschnitt zwischen dem Mantelbereich und dem Bodenabschnitt angeordnet ist, kann in gewohnter Weise am Mantelabschnitt ein Etikett angebracht werden.

Bevorzugte Ausführungsformen folgen aus den übrigen Ansprüchen.

Nach einer bevorzugten Ausführungsform weist der Bodenabschnitt eine konzentrisch angeordnete Wölbung in Richtung auf den Dosierabschnitt auf. Diese Wölbung des Bodenabschnitts bewirkt bei der Benutzung, dass der für das Zusammendrücken des Dosierabschnitts häufig auf den Boden aufgelegte Daumen eines Benutzers automatisch im Bereich der Mitte des Bodenabschnitts zu liegen kommt, wodurch eine gleichmäßige Komprimierung des Dosierabschnitts um den Umfang des im Wesentlichen rotationssymmetrischen Aufbewahrungskörper des Behälters erfolgt.

Nach einer bevorzugten Ausführungsform besitzt der gesamte Dosierabschnitt eine geringere Wandstärke als die erste Wandstärke, und vorzugsweise besitzt der gesamte Dosierabschnitt eine Wandstärke, die um 25% bis 60% gegenüber der ersten Wandstärke reduziert ist. Sofern der gesamte Dosierabschnitt eine geringere Wandstärke besitzt als die erste Wandstärke, kann ein Teil der Bewegung des Faltenbalgs durch die Gelenkstellen, ein anderer Teil der Bewegung des Faltenbalgs aber auch durch eine Biegung der Wandabschnitte des Faltenbalgs zwischen den Gelenkstellen erfolgen. Auf diese Weise werden die Gelenkstellen mechanisch geringer belastet.

Vorzugsweise umfasst der Dosierabschnitt mit einer einzigen Faltung eine erste Gelenkstelle am Mantelabschnitt, eine zweite Gelenkstelle am Bodenabschnitt, einen ersten, ringförmigen Gelenkschenkel, der sich von der ersten Gelenkstelle radial nach innen sowie in einem ersten Winkel relativ zur radialen Richtung erstreckt, einen zweiten, ringförmigen Gelenkschenkel, der sich von der zweiten Gelenkstelle radial nach innen sowie in einem zweiten Winkel relativ zur radialen Richtung erstreckt, sowie eine dritte Gelenkstelle, die den ersten Gelenkschenkel mit dem zweiten Gelenkschenkel verbindet.

Dabei ist es bevorzugt, dass der ersten Gelenkschenkel und der zweite Gelenkschenkel in einer Schnittebene senkrecht zur Längsachse einen im Wesentlichen geradlinigen Verlauf besitzen.

Auf diese Weise ist der Dosierabschnitt ähnlich einer Tellerfeder gebildet, die bei einer Druckbeaufschlagung in Richtung der Längsachse elastisch komprimierbar ist.

Nach einer bevorzugten Ausführungsform besitzen die erste, zweite und dritte Gelenkstelle sowie der zweite Gelenkschenkel eine geringere Wandstärke als die erste Wandstärke, und der erste Gelenkschenkel besitzt eine höhere Wandstärke als der zweite Gelenkschenkel.

Auf diese Weise erfolgt die Bewegung des Dosierabschnitts bei Druck auf den Bodenabschnitt wesentlich im Bereich des zweiten, ringförmigen Gelenkschenkels nahe dem Bodenabschnitt.

Nach einer bevorzugten Ausführungsform ist der erste Winkel geringer als der zweite Winkel.

Auch diese Maßnahme dient dazu, die Verformung des Faltenbalgs bei der Komprimierung des Dosierabschnitts stärker im Bereich der zweiten Gelenkstelle angrenzend an den Bodenabschnitt als im Bereich des ersten Gelenkabschnitts angrenzend an den Mantelabschnitt ablaufen zu lassen.

Dabei ist es bevorzugt, dass der zweite Winkel mehr als doppelt so groß ist wie der erste Winkel, und vorzugsweise beträgt der zweiten Winkel zwischen 30° und 40°.

Nach einer bevorzugten Ausführungsform der Erfindung befindet sich die zweite Gelenkstelle in einem geringeren Abstand zur Längsachse als die erste Gelenkstelle. Auch diese Maßnahme führt wieder dazu, dass die dem Bodenabschnitt zugewandte Hälfte des Faltenbalgs zwischen der dritten Gelenkstelle und der zweiten Gelenkstelle eine stärke Bewegung bei der Komprimierung ausführt als die obere Hälfte des Faltenbalgs, wodurch sich bei einer Komprimierung vor allen Dingen der Dosierabschnitt im Bereich der zweiten Gelenkstelle bewegt.

Vorzugsweise beträgt das Verhältnis zwischen dem Außendurchmesser des Bodenabschnitts zum Innendurchmesser des Behälters im Bereich der dritten Gelenkstelle in etwa dem Verhältnis 2:1.

Auf diese Weise wird im Bereich des Dosierabschnitts eine ausreichend hohe Einfaltung im Bereich des Faltenbalgs erzeugt.

Nach einer bevorzugten Ausführungsform der Erfindung weist der Behälter weiterhin ein Außengewinde am Behälterhalsabschnitt auf. Dieses Außengewinde dient zum Aufschrauben einer Verschlusskappe, mit Hilfe derer sich eine im Behälterhalsabschnitt befindende Ausgabeöffnung zur Abgabe einer sterilen Flüssigkeit verschließen lässt.

Weiterhin bevorzugt ist es, dass der Behälter weiterhin einen Schulterabschnitt zwischen dem Mantelabschnitt und dem Behälterhalsabschnitt aufweist.

Eine derartige Schulterabschnitt kann den Bedienungskomfort erhöhen, indem bei einem Behälter geringer Dimensionen, wie er häufig zur Aufbewahrung und Abgabe von Augentropfen verwendet wird, von einem Benutzer am Schulterabschnitt mit dem Daumen einer Hand und auf der Außenseite des Bodenabschnitts mit einem Finger dieser Hand gehalten werden kann. Bei Behältern mit geringen Abmessungen, wie diese beispielsweise bei Behältern mit einem Inhalt von 5ml gegeben sind, lässt sich auf diese Weise der Behälter bequem halten und der bei der Betätigung des Dosierabschnitts ausgeübte Druck auf den Behälterboden gleichsam durch eine Gegenlager beim gleichzeitigen Halten am Schulterabschnitt auffangen.

Nach einer bevorzugten Ausführungsform der Erfindung weist der Behälter weiterhin eine am Behälterhalsabschnitt vorgesehene, umlaufende Verzahnung auf, die eine Vielzahl von im Wesentlichen tangential verlaufenden Rampenflächen und eine Vielzahl von im Wesentlichen radial verlaufenden Arretierflächen umfasst, die sich jeweils an eine Rampenfläche anschließen.

Die Verzahnung am Behälterhalsabschnitt dient somit dazu, eine Verschlusskappe auf den Aufbewahrungskörper aufzuschrauben, die einen herkömmlichen Originalitätsring besitzt, der über dünnwandige Brückenabschnitte an der Verschlusskappe verbunden ist und über die Verzahnung nach dem Befüllen des Behälters einstückig mit der Verschlusskappe verbunden auf den Aufbewahrungskörper aufgeschraubt werden kann, da der Originalitätsring über die tangential verlaufenden Rampenflächen beim erstmaligen Aufschrauben bewegt werden kann. Beim erstmaligen Öffnen hingegen löst sich der Originalitätsring von der Verschlusskappe, da dieser gegen die Arretierflächen stößt und daran gehindert wird, der Drehbewegung der Verschlusskappe beim Abschrauben zu folgen, wodurch die Brückenabschnitte durchtrennt werden und der Originalitätsring im Bereich der Verzahnung verbleibt, wo er für einen Benutzer ein deutliches optischen Hinweis darauf gibt, dass der Behälter bereits geöffnet wurde und der Inhalt möglicherweise nicht mehr steril sein könnte.

Der Behälter weist vorzugsweise eine zugehörige Verschlusskappe auf, die auf den Aufbewahrungskörper aufschraubbar ist und im aufgeschraubten Zustand den Behälter luftdicht verschließt. Dazu dienen die in der Technik bekannten Maßnahmen wie das Vorsehen einer ringförmigen Dichtung auf der Innenseite der Oberseite der Verschlusskappe, die nach dem Aufschrauben der Verschlusskappe elastisch am Außenumfang einer Ausgabeöffnung des Behälterhalsabschnitts anliegt und den Behälter auf diese Weise dicht verschließt.

### Kurze Beschreibung der Zeichnungen

Nachfolgend wird die Erfindung rein beispielhaft anhand einer Ausführungsform des erfindungsgemäßen Behälters dargestellt. Dabei zeigen:
- Fig. 1: eine dreidimensionale Ansicht des erfindungsgemäßen Behälters;
- Fig. 2: eine Seitenansicht des Behälters nach Fig. 1;
- Fig. 3: eine Schnittansicht des Behälters nach Fig. 2 entlang der Schnittlinie A-A; und
- Fig. 4: eine Draufsicht auf den Behälter nach Fig. 1.

### Wege zur Ausführung der Erfindung

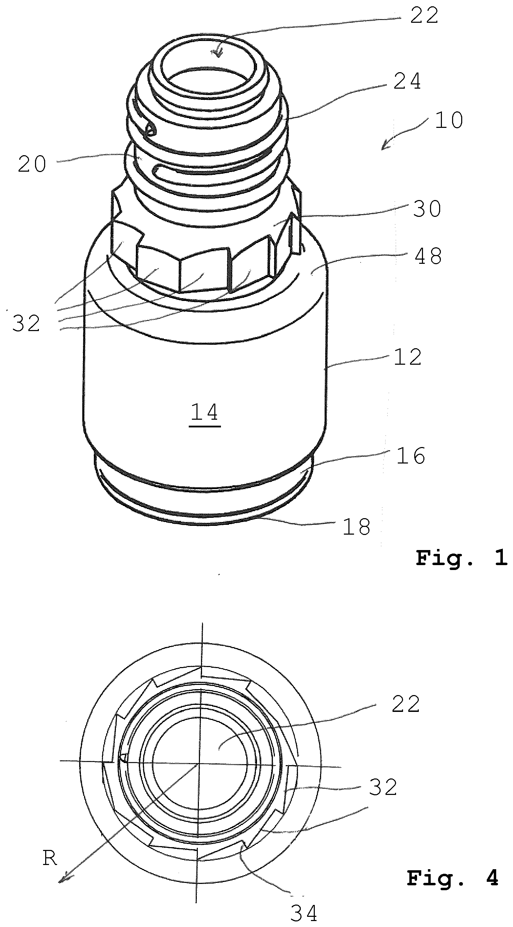

Die Figuren 1 bis 4 zeigen eine Ausführungsform des erfindungsgemäßen Behälters 10 zur dosierten Abgabe einer sterilen Flüssigkeit. Der Behälter ist dabei vorzugsweise so dimensioniert, dass über die später beschriebene Betätigung des Faltenbalgs eine vorgegebene Menge an steriler Flüssigkeit ausgegeben wird.

Der Behälter weist einen Aufbewahrungskörper 12 für die sterile Flüssigkeit auf sowie eine in den Figuren nicht dargestellte Verschlusskappe, die auf den Aufbewahrungskörper aufschraubbar ist. Der Aufbewahrungskörper weist eine Längsachse O auf und ist im Wesentlichen rotationssymmetrisch zur Längsachse O geformt. Senkrecht zur Längsachse verläuft die in Fig. 4 exemplarisch dargestellte radiale Richtung **R.**

Der Aufbewahrungskörper 12 weist einen Mantelabschnitt 14 auf, der im vorliegenden Ausführungsbeispiel im Wesentlichen zylinderförmig ist, aber in gleicher Weise auch kegelförmig ausgestaltet sein kann und eine erste Wandstärke S1 besitzt. An den Mantelabschnitt 14 schließt sich einstückig ein Dosierabschnitt 16 an, und an den Dosierabschnitt 16 schließt sich wiederum einstückig ein Bodenabschnitt 18 an. Desweiteren weist der Aufbewahrungskörper 12 einen Behälterhalsabschnitt 20 auf, der eine Ausgabeöffnung 22 zur Abgabe der sterilen Flüssigkeit umfasst. Am Behälterhalsabschnitt 20 ist ein Außengewinde 24 angeformt, das dem Aufschrauben einer Verschlusskappe (nicht dargestellt) dient. Ein ebenfalls in den Figuren dargestellter, umlaufender Bund 26 dient dazu, den Aufbewahrungskörper insbesondere während des Befüllens mit Flüssigkeit im Rahmen eines industriellen Abfüllungsprozesses halten zu können.

Der Behälter nach der vorliegenden Ausführungsform ist zusätzlich mit einer Originalitätssicherung 28 versehen, die zwischen dem Außengewinde 24 und dem Mantelabschnitt 14 vorgesehen ist und dazu dient, beim erstmaligen Öffnen des Behälters durch Abschrauben der Verschlusskappe einen Originalitätsring, der in üblicher Weise über Brückenstege an der Verschlusskappe angebracht ist, von der Verschlusskappe zu lösen und für einen Benutzer sichtbar zu machen, dass der Behälter bereits geöffnet wurde. Die Ausgestaltung der Originalitätssicherung 28 kann in beliebiger Weise erfolgen. Im vorliegenden Fall ist eine Verzahnung 30 vorgesehen, die eine Mehrzahl von im wesentlichen tangential orientierten Rampenabschnitten 32 und zwischen diesen im wesentlichen radial angeordnete Arretierabschnitte 34 umfasst. Wie aus der Darstellung der Draufsicht nach Fig. 4 ersichtlich ist, kann eine Verschlusskappe in Uhrzeigerrichtung gemeinsam mit dem Originalitätsring erstmalig aufgeschraubt werden, da dieser entsprechend der Wirkung einer Ratsche über die Rampenabschnitte 32 mit der Verschlusskappe mitbewegt wird, während beim ersten Öffnen der Verschlusskappe der Originalitätsring durch die Arretierabschnitte 34 an einer Bewegung entgegen der Uhrzeigerrichtung in Fig. 4 gehindert wird, wodurch die stegförmigen Brückenabschnitte zwischen der Verschlusskappe und dem Originalitätsring reißen und der Originalitätsring nach dem Abschrauben der Verschlusskappe über der Verzahnung verbleibt, während die Verschlusskappe abgeschraubt werden kann. Die in der dargestellten Ausführungsform verwendete Originalitätssicherung kann aber im Rahmen der Erfindung in gleicher Weise durch eine andere in der Technik bekannte Originalitätssicherung ersetzt werden.

Die Dimension der Ausgabeöffnung 22 und insbesondere deren Durchmesser ist bevorzugt an die gebildete Tropfengröße an steriler Flüssigkeit angepasst und kann bei wasserbasierten Flüssigkeiten dabei einen Öffnungsdurchmesser zwischen 8,1mm und 8,3mm besitzen.

Der zwischen dem Mantelabschnitt 14 und dem Bodenabschnitt 18 angeordnete Dosierabschnitt 16 befindet sich an einer Position, wo er nicht mit der Anbringung eines Etiketts am Mantelabschnitt 14 kollidiert und auf diese Weise von einem Benutzer klar erkennbar ist. Der Dosierabschnitt 16 ist als Faltenbalg gestaltet und besitzt eine einzige Einfaltung. Dazu weist der Dosierabschnitt 16 eine erste Gelenkstelle 36, eine zweite Gelenkstelle 38 sowie eine dritte Gelenkstelle 40 auf. Die erste Gelenkstelle 36 stellt den Übergang zwischen dem Mantelabschnitt 14 und dem Dosierabschnitt 16 dar. Die zweite Gelenkstelle 38 stellt den Übergang zwischen dem Bodenabschnitt 18 und dem Dosierabschnitt 16 dar. Die dritte Gelenkstelle 40 ist innerhalb der Einfaltung vorgesehen.

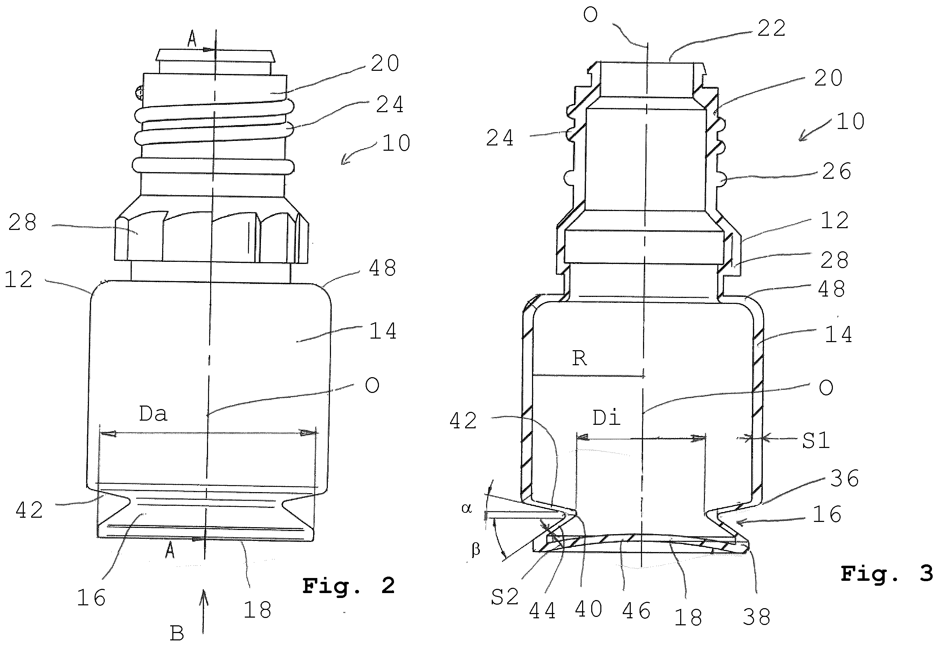

Darüber hinaus ist ein erster Gelenkschenkel 42 vorgesehen, der die erste Gelenkstelle 36 mit der dritten Gelenkstelle 40 verbindet. Ein zweiter Gelenkschenkel 44 verbindet die dritte Gelenkstelle 40 mit der zweiten Gelenkstelle 38. Im vorliegenden Ausführungsbeispiel sind die Gelenkschenkel 42 und 44 ringförmig gestaltet und weisen in der in Fig. 3 dargestellten Schnittebene senkrecht zur Längsachse (O) einen im Wesentlichen geradlinigen Verlauf auf.

Die Wandstärke (S2) des Dosierabschnitts ist dabei um 25% bis 60% gegenüber der ersten Wandstärke (S1) reduziert. Es ist allerdings aber auch möglich, den ersten Gelenkschenkel 42 und den zweiten Gelenkschenkel 44 mit derselben Wandstärke S1 wie den Mantelabschnitt auszugestalten und lediglich im Bereich der Gelenkstellen 36, 38, 40 eine entsprechend verringerte Wandstärke vorzusehen, um in diesen Bereichen eine erhöhte Verformung des Dosierabschnitts 16 zu ermöglichen.

Wie weiterhin in der Fig. 3 ersichtlich ist, sind verschieden große Winkel gebildet, in denen einerseits der erste Gelenkschenkel 42 relativ zur radialen Richtung R verläuft, und in dem andererseits der zweite Gelenkschenkel 44 relativ zur radialen Richtung R verläuft. Im konkreten Ausführungsbeispiel ist der Winkel β zwischen dem zweiten Gelenkschenkel 44 und der radialen Richtung mehr als doppelt so groß wie der Winkel α zwischen dem ersten Gelenkschenkel 42 und der radialen Richtung. Im konkreten Beispiel könnte der Winkel α zwischen 10° und 15° betragen, während der Winkel β zwischen 30° und 40° betragen könnte. Die unterschiedlich großen Winkel dienen dazu, dass bei der später beschriebenen Betätigung des Dosierabschnitts 16 durch einen Druck von außen auf den Bodenabschnitt die Bewegung vor allen Dingen im Bereich der zweiten Gelenkstelle 38 und des zweiten Gelenkschenkels 44 stattfindet. In gleicher Weise ist es nach einer alternativen Ausführungsform aber auch möglich, die Ausgestaltung nach Fig. 3 so abzuwandeln, dass die Betätigung im wesentlichen gleichmäßig über den ersten Gelenkschenkel und den zweiten Gelenkschenkel erfolgt, oder die Betätigung im wesentlichen nur über den ersten Gelenkschenkel erfolgt.

Weiterhin hat es sich als vorteilhaft erwiesen, wenn der Innendurchmesser Di des Behälters im Bereich der dritten Gelenkstelle 40 so gewählt ist, dass dieser etwa halb so groß ist wie der Außendurchmesser Da des Bodenabschnitts 18 im Bereich der zweiten Gelenkstelle 38.

Weiterhin ist es vorteilhaft, wenn sich die zweite Gelenkstelle 38 in einem geringeren radialen Abstand zur Längsachse O befindet als die erste Gelenkstelle 36 zwischen dem Mantelabschnitt 14 und dem Dosierabschnitt 16. Daraus folgt, dass der Radius des Bodenabschnitts 18 geringer ist als der Radius des Mantelabschnitts 14. Diese Geometrie gemeinsam mit dem Verhältnis zwischen den Abmessungen Da:Di hat sich in Bezug auf die bestmögliche Kraftübertragung und Krafteinleitung als besonders vorteilhaft erwiesen. Die Krafteinleitung erfolgt über den Boden 18 in Pfeilrichtung **B.** Durch die zylindersymmetrische Form des Bodens 18 und eine Einwölbung 46 des Bodens in Richtung auf das Behälterinnenvolumen erfolgt die Kraftübertragung im wesentlichen konzentrisch. Die Einwölbung 46 ist vorteilhaft, da der Benutzer bei der Betätigung automatisch in Richtung auf die tiefste Stelle der Einwölbung 46 drückt und sich daher die Konzentrizität der Krafteinleitung verbessert. Für die notwendige Kraftaufwendung ist aber auch der Hebelarm entscheidend. Ein zu kleiner Hebelarm versteift das System und bewirkt eine höhere Betätigungskraft. Wird die Hebellänge allerdings zu groß gewählt, wird in Abhängigkeit von den gewählten Wandstärken der Dosierabschnitt zu weich und es wird nicht mehr ausreichend Kraft in den in Form einer Tellerfeder gestalteten Dosierabschnitt eingeleitet, um diesen zu betätigen. Dies würde dazu führen, dass der benötigte Überdruck zur Bildung eines Tropfens zu gering würde und kein Tropfen aus der als Tropfloch dienenden Ausgabeöffnung 22 austreten würde. Daher hat sich der gewählte Hebelarm im Verhältnis 2:1 zwischen dem Bodenaußendurchmesser Da zum Falteninnendurchmesser Di als sehr geeignet erwiesen, um eine optimale Kraftaufwendung zur Bildung eines Tropfens zu erreichen.

Allerdings kann der Radius des Bodenabschnitts ebenso nach einer alternativen Gestaltung dem Radius des Mantelabschnitts entsprechen.

Der Bodenabschnitt 18 selbst weist ebenfalls eine gewisse Flexibilität auf, so dass auch bei ausreichender Druckbeaufschlagung auf den Boden ein Tropfen freigegeben werden kann, wenn ausschließlich der Boden der Flasche komprimiert wird.

Der erfindungsgemäße Behälter kann aufgrund der Gestaltung des Dosierabschnitts 16 auch aus steiferen Kunststoffmaterialien wie HDPE und PP hergestellt werden, die manchmal aus Gründen der Beständigkeit gegenüber sterilen Flüssigkeiten zum Einsatz kommen.

Ausschlaggebend ist es, zumindest im Bereich der Gelenkstellen eine verringerte Wandstärke für die Funktion der Federung vorzusehen. Es kann sogar vorteilhaft sein, nur die Gelenkstellen mit einer geringeren Wandstärke zu versehen, da sich auf diese Weise das Eindiffundieren von Sauerstoff durch die Außenwand des Behälters reduzieren lässt und es somit bei sauerstoffempfindlichen Produkten zu einem besseren Stabilitätsverhalten kommt. Für einen Benutzer ist aufgrund der Formgebung intuitiv ersichtlich, wie der Behälter zu betätigen ist. Wenn ein Benutzer auf die Mitte des Bodenabschnitts 18 drückt, um den Dosierabschnitt 16 zu komprimieren, hat es sich als vorteilhaft erwiesen, zwischen dem Mantelabschnitt 14 und dem Behälterhalsabschnitt 20 einen Schulterabschnitt 48 vorzusehen, der gleichsam als Gegenlager die Betätigung der Kraftausübung auf den Bodenabschnitt erleichtert.

Der Aufbewahrungskörper 12 des erfindungsgemäßen Behälters kann einstückig aus Kunststoff hergestellt werden, wodurch der Behälter für die Massenfertigung gut geeignet **ist.** Durch die Wahl der Wandstärken im Dosierabschnitt, die Wahl der Winkel zwischen dem ersten Gelenkschenkel zur radialen Richtung, des zweiten Gelenkschenkels zur radialen Richtung sowie des Verhältnisses zwischen dem Bodenaußendurchmesser Da zum Falteninnendurchmesser Di kann eine für den Benutzer immer in etwa gleich hohe Betätigungskraft eingestellt werden, die unabhängig vom Behältervolumen, der sonstigen Wandstärke des Behälters, aber auch vom gewählten Kunststoffmaterial ist.

## Patentansprüche

1. Behälter zur dosierten Abgabe einer sterilen Flüssigkeit, mit
- einem im Wesentlichen rotationssymmetrischen Aufbewahrungskörper (12) aus Kunststoff mit einer Längsachse (O) und einer senkrecht zur Längsachse verlaufenden radialen Richtung (R), wobei der Aufbewahrungskörper (12)
- einen im Wesentlichen zylinderförmigen oder kegelförmigen Mantelabschnitt (14) mit einer ersten Wandstärke (S1),
- einen Dosierabschnitt (16),
- einen Bodenabschnitt (18) und
- einen Behälterhalsabschnitt (20) umfasst, wobei
- der Dosierabschnitt (16) zwischen dem Mantelabschnitt (14) und dem Bodenabschnitt (18) angeordnet ist, und
- als Faltenbalg umfassend Gelenkstellen (36, 38, 40) ausgestaltet ist,
- wobei der Dosierabschnitt (16) in Richtung der Längsachse elastisch komprimierbar ist;
**dadurch gekennzeichnet, dass**
- der Faltenbalg eine einzige Faltung radial nach innen aufweist;
- der Behälter zusätzlich mit einer Originalitätssicherung (28) versehen ist; und
- die Wandstärke (S2) des Dosierabschnitts (16) zumindest im Bereich der Gelenkstellen (36, 38, 40) um 25% bis 60% gegenüber der ersten Wandstärke S1 reduziert ist.

2. Behälter nach Anspruch 1,
**dadurch gekennzeichnet, dass**
der Bodenabschnitt (18) eine konzentrisch angeordnete Wölbung (46) in Richtung auf den Dosierabschnitt (16) aufweist.

3. Behälter nach Anspruch 1 oder Anspruch 2,
**dadurch gekennzeichnet, dass**
der gesamte Dosierabschnitt (16) eine geringere Wandstärke (S2) besitzt als die erste Wandstärke (S1), vorzugsweise der gesamte Dosierabschnitt (16) eine Wandstärke besitzt, die um 25% bis 60% gegenüber der ersten Wandstärke (S1) reduziert ist.

4. Behälter nach einem der Ansprüche 1 bis 3,
**dadurch gekennzeichnet, dass**
- die einzige Faltung umfasst:
- eine erste Gelenkstelle (36) am Mantelabschnitt (14),
- eine zweite Gelenkstelle (38) am Bodenabschnitt (18),
- einen ersten, ringförmigen Gelenkschenkel (42) der sich von der ersten Gelenkstelle (36) radial nach innen sowie in einem ersten Winkel (α) relativ zur radialen Richtung (R) erstreckt,
- einen zweiten, ringförmigen Gelenkschenkel (44), der sich von der zweiten Gelenkstelle (38) radial nach innen sowie in einem zweiten Winkel (β) relativ zur radialen Richtung (R) erstreckt; sowie
- eine dritte Gelenkstelle (40), die den ersten Gelenkschenkel (42) mit dem zweiten Gelenkschenkel (44) verbindet.

5. Behälter nach Anspruch 4,
**dadurch gekennzeichnet, dass**
die erste, zweite und dritte Gelenkstelle (36, 38, 40) sowie der zweite Gelenkschenkel (44) eine geringere Wandstärke besitzen als die erste Wandstärke (S1), und der erste Gelenkschenkel (42) eine höhere Wandstärke besitzt als der zweite Gelenkschenkel (44).

6. Behälter nach Anspruch 4 oder Anspruch 5,
**dadurch gekennzeichnet, dass**
der erste Gelenkschenkel (42) und der zweite Gelenkschenkel (44) in einer Schnittebene senkrecht zur Längsachse (O) einen im Wesentlichen geradlinigen Verlauf besitzen.

7. Behälter nach Anspruch 6,
**dadurch gekennzeichnet, dass**
der erste Winkel (α) geringer ist als der zweite Winkel (β).

8. Behälter nach Anspruch 7,
**dadurch gekennzeichnet, dass**
der zweite Winkel (β) mehr als doppelt so groß ist wie der erste Winkel (α), und vorzugsweise der zweite Winkel (β) zwischen 30° und 40° beträgt.

9. Behälter nach einem der Ansprüche 4 bis 8,
**dadurch gekennzeichnet, dass**
sich die zweite Gelenkstelle (38) in einem geringeren radialen Abstand zur Längsachse (O) befindet als die erste Gelenkstelle (36).

10. Behälter nach einem der Ansprüche 4 bis 9,
**dadurch gekennzeichnet, dass**
das Verhältnis zwischen dem Außendurchmesser (Da) des Bodenabschnitts (18) zum Innendurchmesser (Di) des Behälters im Bereich der dritten Gelenkstelle (40) in etwa dem Verhältnis 2:1 entspricht.

11. Behälter nach einem der vorhergehenden Ansprüche, weiter umfassend ein Außengewinde (24) am Behälterhalsabschnitt (20).

12. Behälter nach einem der vorhergehenden Ansprüche, weiter umfassend einen Schulterabschnitt (48) zwischen dem Mantelabschnitt (14) und dem Behälterhalsabschnitt (20).

13. Behälter nach einem der vorhergehenden Ansprüche, weiter umfassend eine am Behälterhalsabschnitt (20) vorgesehene, umlaufende Verzahnung (30) umfassend eine Vielzahl von im Wesentlichen tangential verlaufenden Rampenflächen (32) und eine Vielzahl von im Wesentlichen radial verlaufenden Arretierflächen (34), die sich jeweils an eine Rampenfläche (32) anschließen.

14. Behälter nach einem der vorhergehenden Ansprüche, weiter umfassend eine zugehörige Verschlusskappe, die auf den Aufbewahrungskörper (12) aufschraubbar ist und im aufgeschraubten Zustand den Behälter dicht verschließt.

## Claims

1. Container for dosed dispensing of a sterile liquid, having
- a substantially rotationally symmetrical storage body (12) made of plastics material having a longitudinal axis (O) and a radial direction (R) extending perpendicularly to the longitudinal axis, wherein the storage body (12) comprises
- a substantially cylindrical or conical sheath portion (14) having a first wall thickness (S1),
- a dosing portion (16),
- a base portion (18) and
- a container neck portion (20), wherein
- the dosing portion (16) is arranged between the sheath portion (14) and the base portion (18), and
- is designed as joints (36, 38, 40) comprising bellows,
- wherein the dosing portion (16) can be elastically compressed in the direction of the longitudinal axis;
**characterised in that**
- the bellows have a single radially inward fold;
- the container is additionally provided with a tamper-evident seal (28); and
- the wall thickness (S2) of the metering portion (16) is reduced at least in the region of the joints (36, 38, 40) by 25% to 60% relative to the first wall thickness S1.

2. Container according to claim 1,
**characterised in that**
the base portion (18) has a concentrically arranged bulge (46) in the direction of the dosing portion (16).

3. Container according to claim 1 or claim 2,
**characterised in that**
the entire dosing portion (16) has a lower wall thickness (S2) than the first wall thickness (S1), preferably the entire dosing portion (16) has a wall thickness that is reduced by 25% to 60% relative to the first wall thickness (S1).

4. Container according to any of claims 1 to 3,
**characterised in that**
- the single fold comprises:
- a first joint (36) on the sheath portion (14),
- a second joint (38) on the base portion (18),
- a first, annular joint limb (42) that extends from the first joint (36) radially inwards and at a first angle (α) relative to the radial direction (R),
- a second, annular joint limb (44) that extends from the second joint (38) radially inwards and at a second angle (β) relative to the radial direction (R); and
- a third joint limb (40) that connects the first joint limb (42) to the second joint limb (44).

5. Container according to claim 4,
**characterised in that**
the first, second and third joint (36, 38, 40) and the second joint limb (44) have a lower wall thickness than the first wall thickness (S1), and the first joint limb (42) has a higher wall thickness than the second joint limb (44).

6. Container according to claim 4 or claim 5,
**characterised in that**
the first joint limb (42) and the second joint limb (44) have a substantially rectilinear course in a sectional plane perpendicular to the longitudinal axis (O).

7. Container according to claim 6,
**characterised in that**
the first angle (α) is less than the second angle (β).

8. Container according to claim 7,
**characterised in that**
the second angle (β) is more than twice as large as the first angle (α), and preferably the second angle (β) is between 30° and 40°.

9. Container according to any of claims 4 to 8,
**characterised in that**
the second joint (38) is at a shorter radial distance from the longitudinal axis (O) than the first joint (36).

10. Container according to any of claims 4 to 9,
**characterised in that**
the ratio of the outer diameter (Da) of the base portion (18) to the inner diameter (Di) of the container in the region of the third joint (40) approximately corresponds to the ratio 2:1.

11. Container according to any of the preceding claims, further comprising an external thread (24) on the container neck portion (20).

12. Container according to any of the preceding claims, further comprising a shoulder portion (48) between the sheath portion (14) and the container neck portion (20).

13. Container according to any of the preceding claims, further comprising circumferential toothing (30) provided on the container neck portion (20), comprising a plurality of substantially tangentially extending ramp surfaces (32) and a plurality of substantially radially extending locking surfaces (34) that each adjoin a ramp surface (32).

14. Container according to any of the preceding claims, further comprising an associated closure cap that can be screwed onto the storage body (12) and that sealingly closes the container in the screwed-on state.

## Revendications

1. Récipient pour la distribution dosée d'un fluide stérile, avec
- un corps de stockage (12) sensiblement symétrique en rotation en matière plastique présentant un axe longitudinal (O) et une direction radiale (R) perpendiculaire à l'axe longitudinal, dans lequel le corps de stockage (12)
- comprend une section de gaine (14) sensiblement cylindrique ou conique présentant une première épaisseur (S1) de paroi,
- une section de dosage (16),
- une section de fond (18) et
- une section de col (20) de récipient, dans lequel
- la section de dosage (16) est disposée entre la section de gaine (14) et la section de fond (18), et
- est conçue en tant que soufflet comprenant des points d'articulation (36, 38, 40),
- dans lequel la section de dosage (16) est compressible élastiquement dans la direction de l'axe longitudinal ;
**caractérisé en ce que**
- le soufflet présente un seul pli radial vers l'intérieur ;
- le récipient est en outre pourvu d'une sécurité antivol (28) ; et
- l'épaisseur (S2) de paroi de la section de dosage (16) est réduite d'au moins 25 % à 60 % par rapport à la première épaisseur S1 de paroi au niveau des points d'articulation (36, 38, 40).

2. Récipient selon la revendication 1,
**caractérisé en ce que**
la section de fond (18) présente une courbure (46) disposée concentriquement en direction de la section de dosage (16).

3. Récipient selon la revendication 1 ou la revendication 2,
**caractérisé en ce que**
toute la section de dosage (16) présente une épaisseur (S2) de paroi inférieure à la première épaisseur (S1) de paroi, de préférence toute la section de dosage (16) présente une épaisseur de paroi qui est réduite de 25 % à 60 % par rapport à la première épaisseur (S1) de paroi.

4. Procédé selon l'une quelconque des revendications 1 à 3,
**caractérisé en ce que**
- le seul pli comprend :
- un premier point d'articulation (36) sur la section de gaine (14),
- un deuxième point d'articulation (38) sur la section de fond (18),
- une première branche articulée (42) en forme d'anneau qui s'étend radialement vers l'intérieur à partir du premier point d'articulation (36) ainsi qu'à un premier angle (α) par rapport à la direction radiale (R),
- une deuxième branche articulée (44) en forme d'anneau qui s'étend radialement vers l'intérieur à partir du deuxième point d'articulation (38) ainsi qu'à un deuxième angle (β) par rapport à la direction radiale (R) ; et
- un troisième point d'articulation (40) qui relie la première branche articulée (42) à la deuxième branche articulée (44).

5. Récipient selon la revendication 4,
**caractérisé en ce que**
les premier, deuxième et troisième points d'articulation (36, 38, 40) ainsi que la deuxième branche articulée (44) présentent une épaisseur de paroi inférieure à la première épaisseur (S1) de paroi, et la première branche articulée (42) présente une épaisseur de paroi supérieure à la deuxième branche articulée (44).

6. Récipient selon la revendication 4 ou la revendication 5,
**caractérisé en ce que**
la première branche articulée (42) et la deuxième branche articulée (44) présentent un tracé sensiblement rectiligne dans un plan de coupe perpendiculaire à l'axe longitudinal (O).

7. Récipient selon la revendication 6,
**caractérisé en ce que**
le premier angle (α) est inférieur au deuxième angle (β).

8. Récipient selon la revendication 7,
**caractérisé en ce que**
le deuxième angle (β) est deux fois supérieur au premier angle (α), et de préférence le deuxième angle (β) est compris entre 30° et 40°.

9. Procédé selon l'une quelconque des revendications 4 à 8,
**caractérisé en ce que**
le deuxième point d'articulation (38) se trouve à une distance radiale par rapport à l'axe longitudinal (O) plus petite que le premier point d'articulation (36).

10. Procédé selon l'une quelconque des revendications 4 à 9,
**caractérisé en ce que**
le rapport entre le diamètre extérieur (Da) de la section de fond (18) et le diamètre intérieur (Di) du récipient au niveau du troisième point d'articulation (40) correspond à peu près au rapport 2:1.

11. Récipient selon l'une quelconque des revendications précédentes, comprenant en outre un filetage externe (24) sur la section de col (20) de récipient.

12. Récipient selon l'une quelconque des revendications précédentes, comprenant en outre une section d'épaulement (48) entre la section de gaine (14) et la section de col (20) de récipient.

13. Récipient selon l'une quelconque des revendications précédentes, comprenant en outre une denture périphérique (30) prévue sur la section de col (20) de récipient comprenant une pluralité de surfaces (32) de rampe sensiblement tangentielles et une pluralité de surfaces d'arrêt (34) sensiblement radiales, qui se raccordent respectivement à une surface (32) de rampe.

14. Récipient selon l'une quelconque des revendications précédentes, comprenant en outre un capuchon de fermeture correspondant qui peut être vissé sur le corps de stockage (12) et ferme hermétiquement le récipient à l'état vissé.
